# EUROPEAN PATENT SPECIFICATION

(11) **EP 3 020 080 B1**
(45) Date of publication and mention of the grant of the patent: **07.08.2019**
(21) Application number: 14822324.1
(22) Date of filing: 11.07.2014
(51) Int. Cl.: H01M 2/02, A61N 1/36, A61N 1/375, A61N 1/378, H01M 2/10, H01M 2/20, H01M 10/04, H01M 10/42, A61N 1/39

(54) **BATTERY AND ELECTRONICS INTEGRATION IN AN IMPLANTABLE MEDICAL DEVICE**
BATTERIEN- UND ELEKTRONIKINTEGRATION IN EINE IMPLANTIERBARE MEDIZINISCHE VORRICHTUNG
BATTERIE ET INTÉGRATION DE COMPOSANTS ÉLECTRONIQUES DANS UN DISPOSITIF MÉDICAL IMPLANTABLE

(30) Priority: 11.07.2013 US 201361844880 P
(43) Date of publication of application: 18.05.2016
(73) Proprietor: Newpace Ltd., 3088900 Caesarea (IL)
(72) Inventor: STROMMER, Gera, 3475950 Haifa (IL); BRODER, Avi, 49712 Petach Tikva (IL); MOCHA, Moti, 5020000 Beit Dagan (IL); FISHEL, Robert, Delray Beach, FL 33483 (US)
(74) Representative: Schuhmann, Albrecht
(86) International application number: PCT/IL2014/050629
(87) International publication number: WO 2015/004673

(56) References cited:
- EP-A1- 2 422 842
- WO-A1-2012/138782
- WO-A2-2014/089299
- US-A- 5 645 586
- US-A1- 2004 176 817
- US-A1- 2004 176 817
- US-A1- 2006 212 087
- US-A1- 2006 217 779
- US-A1- 2008 077 219
- US-A1- 2008 093 110
- US-A1- 2008 167 702
- US-A1- 2009 266 573
- US-B1- 6 658 296

## Description

### FIELD OF THE DISCLOSED TECHNIQUE

The disclosed technique relates to battery and electronics integration, in general, and to methods and systems for integrating a battery and electronics in flexible implantable medical devices as well as non-implanted medical devices, in particular.

### BACKGROUND OF THE DISCLOSED TECHNIQUE

Implantable medical devices, such as pacemakers, defibrillators, brain stimulators, pain relief stimulators, sleep apnea stimulators, other stimulation devices and the like, require a source of power to function and operate. The source of power is usually a battery which is commonly contained in a can together with electronic components. The can is usually attached to another part of the implantable medical device which delivers some kind of therapy to a patient based on electrical impulses. As such devices may be typically worn or carried by patients for years or even decades the battery is usually implanted in the patient as part of the implantable medical device and is typically integrated into the device and not removable. When the battery dies and needs replacement, the patient must undergo surgery to remove the battery and replace it with a new one. In some devices, the entire device needs to be replaced as the battery is not a separately replaceable component. Thus the entire medical device requires replacement at the time of battery depletion. State of the art batteries used in such devices may last anywhere up to 5-7 years. However a patient who receives a pacemaker or defibrillator early in his or her life, such at age 40, and lives into his 80s may have to undergo multiple surgeries just to replace the battery of his pacemaker or defibrillator.

In many implantable medical devices, a part of the device, such as an electrical lead, may be positioned apart from the can containing the battery and electronics accordingly. For example, in prior art pacemakers, electrical leads which are used to both measure the heart's electrical activity and also provide electrical stimulation to the heart are placed in a different location than the can which houses the battery as well as electronics for controlling the pacemaker. The electrical leads are usually positioned within the heart, whereas the can may be positioned under the collarbone. Implanting the pacemaker requires major surgery as the electrical leads need to be positioned within the heart of a patient. In addition, an incision needs to be made to position the can in the body of the patient. At period intervals typically ranging from 5 to 7 years, the patient will have to undergo surgery to enable access to the can where the old battery is. The can is then replaced by removing it and inserting a new can, containing a new battery, in the patient. In addition, if any issues or problems ever occur with the electrical leads, the patient will again have to undergo major surgery to fix, repair or replace the electrical leads within the heart. It is noted that removing old electrical leads from the heart may be a complex medical procedure which can cause additional complications. In some cases, the old electrical leads may be left in the heart and new electrical leads are implanted next to the old ones. The can, which in prior art pacemakers is substantially bulky, is usually positioned in the body such that the patient will not be impaired with regard to physical movement and also to reduce any discomfort in the patient due to the positioning of the can. The patient though may suffer from discomfort in the tissue area that surrounds the can if a significant force is placed on the area, such as by getting hit in the area or falling on the area. In addition, thin patients or patients with limited amounts of subcutaneous tissue may also risk erosion of the device, for example the can, through the skin.

The integration of the power source with the other parts of an implantable medical device, such as the electrical leads, into a single unit in order that the can does not need to be separated from the electrical leads would make such an implantable medical device easier to handle and would simplify the surgery required to insert and remove the device in a patient. Such a unit could also include at least one electronic circuit or a series of electronic circuits as well as at least one capacitor. However replacing the battery of such a device every few years would still require the patient to undergo surgery. Such a device is described in U.S. Patent No. 7,985,500 to Root et al., entitled "Method and apparatus for flexible battery for implantable device," which is directed to an apparatus for storing energy, the apparatus having a first portion comprising a flexible substrate containing a polymer electrolyte and a second portion adapted to provide a conformable housing surrounding the first portion. The apparatus is adapted to provide a source of energy to an implantable device. The apparatus with the implantable device forms a flexible implantable device capable of traversing the circulatory system of a body with minimal obstruction of flow within the circulatory system. In other embodiments of the apparatus to Root, the apparatus comprises at least one single cell contained within a flexible housing. Such an apparatus is adaptable to provide a source of energy to an implantable device. The apparatus can also contain both a sensor and a power source within the flexible housing. The housing can include an anchoring mechanism for anchoring the device during implantation within the body. The apparatus can also include a series of smaller battery cells attached by flexible conductive interconnects that are further contained within the conformable housing capable of traversing the circulatory system of the body.

What is needed then is an implantable medical device having a structure that incorporates the power source and electronic components, thus simplifying its placement in a patient, yet which also allows the power source to be easily replaced requiring only minor, less-invasive surgery. In addition, such a device should not impair a patient's movement at all and should cause no discomfort to the patient during their daily routine and activities.

Implantable medical device structures which incorporate a power source and electronic components are known in the art. US patent application publication no. 2004/0176817 A1 to Wahlstrand et al., entitled "Modular Implantable Medical Device", is directed to an implantable medical device for implantation in the head of a patient. The implantable medical device has a plurality of interconnected modules which may be covered by an overmold or which may be partially covered by the overmold. The module(s) covered by the overmold may be implanted between the cranium and scalp, while the module(s) partially covered by the overmold may be placed at least partially into a recess in the cranium.

US patent no. 6,658,296 B1 to Wong et al., entitled "Implantable Cardioverter Defibrillator having an Articulated Flexible Circuit Element and Method of Manufacturing", is directed to an implantable cardiac rhythm management device having a flexible circuit sheet with a number of connected sheet portions and a number of conductive traces extending between different sheet portions. A plurality of device components is attached to the sheet on different sheet portions. The sheet is articulated at fold lines between the sheet portions and is folded so that at least some of the sheet portions occupy different planes.

European patent application publication no. 2 422 842 A1 to Second Sight Medical Products, Inc., entitled "Package for an implantable neural stimulation device", is directed to an improved hermetic package for implantation in the human body. The implantable device includes an electrically non-conductive substrate including electrically conductive vias through the substrate. A circuit is flip-chip bonded to a subset of the vias. A second circuit is wire bonded to another subset of the vias. Finally, a cover is bonded to the substrate such that the cover, substrate and vias form a hermetic package.

US patent application publication no. 2009/0266573 A1 to Engmark et al., entitled "Assembly Method for Implantable Medical Device", is directed to an implantable medical device (IMD) having a hermetic housing formed from a case and a cover each having an exterior surface and an interior surface. A component of the IMD is mounted to the interior surface of the cover and has an electrical contact. A hybrid circuit is assembled in the case. The electrical contact of the component is electrically coupled to the hybrid circuit assembled in the case.

US patent application publication no. 2006/0212087 A1 to Haller et al., entitled "Implantable Stimulator", is directed to implantable stimulators and methods of their use for stimulating a stimulation site within a patient. The implantable stimulator includes an electronic module configured to generate stimulation and a housing configured to house the electronic module. The housing has a shape allowing the stimulator and a surgical device to be accommodated together within an insertion tool used to insert the stimulator into the patient.

### SUMMARY OF THE DISCLOSED TECHNIQUE

It is an object of the disclosed technique to provide a novel battery and electronics configuration, enabling the battery to be integrated into any implantable medical device having a flexible string-like or snake-like shape or form. In accordance with the disclosed technique, there is thus provided an encapsulation configuration for electronic components in a flexible implantable medical device, including a plurality of circuit boards and a plurality of connection cables. Each one of the circuit boards includes at least one electronics component. The encapsulation configuration is characterized in that each one of the circuit boards has a generally circular shape and each one of the connection cables electrically couples adjacent ones of the circuit boards alternatively at opposite ends. Each one the circuit boards is folded over an adjacent one of the circuit boards in a pleated manner, thereby giving the encapsulation configuration an accordion-like shape.

In accordance with another embodiment of the disclosed technique, the encapsulation configuration further includes a flat circuit board, which includes a plurality of electronics components. The flat circuit board has a generally rectangular shape and the electronics components are positioned on both sides of the flat circuit board. Taller ones of the electronics components are positioned closer to the center of the flat circuit board and shorter ones of the electronics components are positioned closer to the edges of the flat circuit board, thereby achieving optimal volume consumption in the flat circuit board. A first one of the circuit boards is coupled with the flat circuit board with a connection cable and the encapsulation configuration has a cylindrical shape.

### BRIEF DESCRIPTION OF THE DRAWINGS

The disclosed technique will be understood and appreciated more fully from the following detailed description taken in conjunction with the drawings in which:
Figure 1 is a schematic illustration of a first battery configuration, constructed and operative in accordance with an embodiment of the disclosed technique;
Figure 2 is a schematic illustration of a second battery configuration, constructed and operative in accordance with another embodiment of the disclosed technique;
Figure 3 is a schematic illustration of a third battery configuration, constructed and operative in accordance with a further embodiment of the disclosed technique;
Figure 4 is a schematic illustration of a fourth battery configuration, constructed and operative in accordance with another embodiment of the disclosed technique;
Figure 5 is a schematic illustration of an implantable medical device having a flexible string shape, constructed and operative in accordance with a further embodiment of the disclosed technique;
Figure 6 is a schematic illustration of a battery integrated into the implantable medical device of Figure 5, constructed and operative in accordance with another embodiment of the disclosed technique;
Figure 7 is a schematic illustration and close-up of an implantable medical device with a removable battery, constructed and operative in accordance with a further embodiment of the disclosed technique;
Figure 8 is a schematic illustration of various possible shapes for an implantable medical device having a flexible string shape, constructed and operative in accordance with another embodiment of the disclosed technique;
Figure 9A is a schematic illustration of an encapsulation configuration for electronic components in a flexible implantable medical device, shown in an unfolded view, constructed and operative in accordance with a further embodiment of the disclosed technique;
Figure 9B is an image of electronic components in the encapsulation configuration of Figure 9A, constructed and operative in accordance with another embodiment of the disclosed technique;
Figures 10A and 10B are schematic illustrations of the encapsulation configuration of Figures 9A and 9B shown in a folded view, constructed and operative in accordance with a further embodiment of the disclosed technique;
Figures 10C and 10D are schematic illustrations of another encapsulation configuration for electronic components in a flexible implantable medical device, constructed and operative in accordance with another embodiment of the disclosed technique;
Figures 10E and 10F are schematic illustrations of a further encapsulation configuration for electronic components in a flexible implantable medical device, constructed and operative in accordance with a further embodiment of the disclosed technique;
Figure 11 is a schematic illustration of a single flat battery chip, shown in an exploded view, constructed and operative in accordance with another embodiment of the disclosed technique;
Figure 12 is a schematic illustration of a plurality of single flat battery chips of Figure 11, showing how the cathodes and anodes of each single flat battery chip are coupled together, constructed and operative in accordance with a further embodiment of the disclosed technique; and
Figure 13 is a schematic illustration of the plurality of single flat battery chips of Figure 12 fully assembled into a battery, constructed and operative in accordance with another embodiment of the disclosed technique.

### DETAILED DESCRIPTION OF THE EMBODIMENTS

The disclosed technique overcomes the disadvantages of the prior art by providing a novel battery and electronics configuration, enabling the battery to be integrated into any implantable medical device having a flexible string-like or snake-like shape or form. The disclosed technique also provides for a novel encapsulation configuration for electronic components in an implantable medical device to be positioned and fitted compactly in such a device. The disclosed technique further provides for a novel battery which includes a plurality of flat high power single battery cells coupled together to form a battery unit. The disclosed technique can also be used in medical devices which are not implanted in a patient. The battery may be rigid or flexible, yet in either configuration, it enables the implantable medical device a significant amount of flexibility. The battery configuration enables the implantable medical device to include only one part which has a string-like shape, thus simplifying its insertion and placement within a patient. In addition, the battery configuration (along with the other components of an implantable medical device having a string-like shape) enables the battery to be easily and quickly removed and inserted after the implantable medical device has already been implanted in a patient without the need to remove the device itself, or without the need to remove a cover, frame or sheath positioned inside the patient which houses the implantable medical device. That being said, according to the disclosed technique, the whole implantable medical device can alternatively be removed through a small incision in the skin requiring only minor, less-invasive surgery due to its low profile and flexible string shape. The old battery of the device can then be removed and replaced with a new battery. The implantable medical device can then be reinserted into the patient via the small incision, which can then be simply sutured up. Thus the battery configuration of the disclosed technique enables the power source in an implantable medical device to be changed and replaced without requiring major surgery. It is noted that the battery configuration can be used in implantable medical devices which are inserted endovascularly as well as subcutaneously. In particular, according to the disclosed technique, implantable medical devices inserted subcutaneously having a flexible string shape can be easily removed and inserted due to the presence of a semi-rigid sheath which encapsulates the device, including its power source. As described below, the sheath can be left in the body of a patient, while the core of the device including the power source, the other parts of the implantable medical device or both can be easily removed. A core including a new power source or other components of the implantable medical device can then be reinserted into the sheath. In addition, as mentioned above, the disclosed technique can be used in other medical devices which are not implanted but are placed on the body of a patient and have a string-like shape. This may include stimulation devices which include replaceable sticky patches that are placed on the body. Besides holding the device, these patches provide electrical impulses to the patient and can be coupled via a device having a string-like shape, which includes a power source and necessary electronics for providing the electrical impulses to the patches. The power source itself may be embodied as a single battery, a plurality of batteries or a plurality of batteries using hybrid battery chemistry.

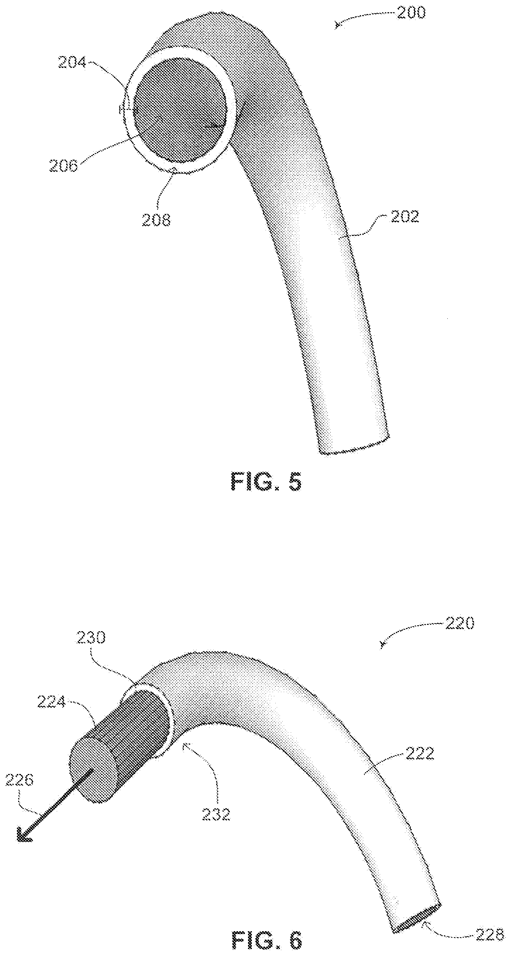

As mentioned above, many implantable medical devices require a power source for delivering electrical pulses to various parts of the body. Such electrical pulses can be used to regulate various organs and systems of the body. Prior art implantable medical devices usually separate the power source from the electrodes which actually deliver the electrical pulses to at least one location in the body, thus resulting in an implantable medical device having at least two parts placed in different positions within a patient. The disclosed technique provides for a battery configuration enabling the power source to be integrated into the same housing as the electrodes, thus forming an implantable medical device having only one part and being essentially unitary. In general, the disclosed technique relates to any implantable medical device having a flexible string shape. Examples of such string shapes are shown below in Figures 5, 6, 7 and 8. In addition, other examples of such a device can be found in U.S. Provisional Patent Application No. 61/728,897 and U.S. Provisional Patent Application No. 61/765,195, both to the same inventors of the current patent application. These patent applications relate to a pacemaker and defibrillator having a string shape which can be implanted in a patient subcutaneously. Other types of implantable medical devices having a string shape include a string-shaped pacemaker, a string-shaped defibrillator or ICD (implantable cardioverter defibrillator), a string-shaped heart device combining pacing and defibrillation functions (i.e., a device similar to prior art implanted cardiac resynchronization devices (CRT-Ds) except in the shape of a string and not having a separate can and leads design), a string-shaped spine stimulator, a string-shaped neurostimulation device for pain management, a string-shaped brain stimulator for deep brain stimulation and for aiding patients with sleep apnea, a string-shaped brain pacemaker and the like. The disclosed technique can also be used for medical devices having the above mentioned string shape, such as implantable pain control devices, implantable bladder stimulator devices, implantable sphincter control devices, implantable neurostimulator devices, implantable drug delivery devices and implantable monitoring devices.

In general, the terms "string shape," "flexible string shape" and "string-like shape" as used herein with reference to a medical device refer to any type of medical device having the following characteristics:
- can provide any known stimulation type therapy, wherein an organ, a muscle or a part thereof, is stimulated via electrical impulses;
- is embodied as a single unit, including the power source, electrodes and any other electronics (such as a CPU, at least one capacitor and the like) required to provide the electrical impulses as stimulation (thus not having a separate can and leads configuration as described in the prior art);
- can be positioned inside a patient endovascularly, subcutaneously, internally, percutaneously and the like, yet can also be positioned externally to (i.e., on the outer surface of) the patient's body;
- can be positioned inside a semi-rigid sheath such that it can be easily inserted and removed from the sheath, even if the sheath is implanted inside a patient (i.e., sheath remains implanted while ICD is removable with respect to the position of the sheath);
- has a generally tubular or cylindrical shape with a cross-sectional shape having any known curvature. For example, the cross-sectional shape may be a circle, an ellipse, a polygon, a closed curve and the like. The cross-sectional shape may also be any conic section having an eccentricity ranging from 0 to 1. In addition, the cross-sectional shape may vary or change over length, being different at a distal end as compared to a proximal end of a medical device.

An implantable medical device having a string shape according to the disclosed technique integrates the full functionality of a medical device used for stimulating internal organs, via the administration of electrical pulses, into a single flexible structure having the shape of a flexible string. Such a structure will include at least one sensing electrode, for acquiring and measuring a biological signal from an organ of interest (such as the heart, the brain, the lungs and the like), at least one signal delivery electrode, for delivering an electrical pulse as a way to synchronize the organ of interest or provide a therapy to it, a processor, for analyzing the acquired and measured biological signal and determining what type of electrical signal should be administered to the organ of interest (for example, the strength of the electrical pulse, the frequency or rate at which the electrical pulse should be delivered, the total amount of time the electrical pulse should be delivered and the like) and a power source, such as a battery, for providing the implantable medical device with a substantially continuous supply of power. In some structures, a capacitor and an electronic circuit may also be included in order to generate and store a high voltage for generating a high current electric shock, as is needed in the case of defibrillation. It is noted that the capacitor and electronic circuit may be embodied as a plurality of capacitors coupled together via coils, resistors, transistors, diodes and/or other appropriate electronic components depending on the voltage, energy and waveform required. The coupling of the capacitors can also be either in series, parallel or a mixture of the two. This is a matter of design choice depending on which internal organ or organs are to be stimulated and what kind of stimulation therapy is to be applied to the organ or organs. In such structures the power source is also used in the building up of such a high voltage electrical pulse. Such a structure is novel in that all the components of the implantable medical device are integrated into a single structure or a core structure. This is unlike prior art implantable medical devices which include a can and a pair of leads, where the can is used to house the processor, the power source and the capacitor (if required) while the pair of leads house both the sensing and signal delivery electrodes. In such prior art devices, the pair of leads are coupled with the can, and particularly with the internal components housed in the can. As mentioned above, the power source may be embodied as a single battery, a plurality of batteries or a plurality of batteries using hybrid battery technology.

In general, the power source in implantable medical devices requires the most amount of volume relative to the volume occupied by other components and according to the prior art thus requires a separate can in which it is housed. According to the disclosed technique, the power source of an implantable medical device is integrated into the same housing which includes the sensing and signal delivery electrodes along with the processor, and if required, the capacitor as well, thus forming a core structure. As mentioned above, the capacitor may be embodied as a plurality of capacitors coupled together with coils and other appropriate electronic components in series, in parallel or in both. As described, the plurality of capacitors and electronic components, according to the disclosed technique form part of the core structure. Thus the disclosed technique eliminates the need for an implantable medical device to have a can and leads design, wherein the can may be implanted in one part of a patient, with the leads implanted in another part of the patient and the two elements (can and leads) are coupled together into a single solitary device. It is noted that in another embodiment of the disclosed technique, a plurality of string-shaped implantable medical devices can be coupled together (for example, in series), thus forming a multiple string-shaped implantable medical device. Such a device might be used when the implantable medical device is to serve multiple functions, such as acting as a pacemaker as well as a defibrillator. In such a case, the various functions may be split amongst the implantable medical devices. For example, a first string-shaped implantable medical device might include electronics for enabling the pacing function whereas a second string-shaped implantable medical device might include electronics for enabling the defibrillation function. Both string-shaped implantable medical devices are coupled together, however, and thus function together as one implantable medical device. In another embodiment, the electronics for both the first string-shaped implantable medical device and the second string-shaped implantable medical device may be inserted in only one of the string-shaped implantable medical devices. Thus, the two implantable medical devices each serve a different function (one for the pacing function and another for the defibrillation function) yet the electronics are placed in only one of the implantable medical devices.

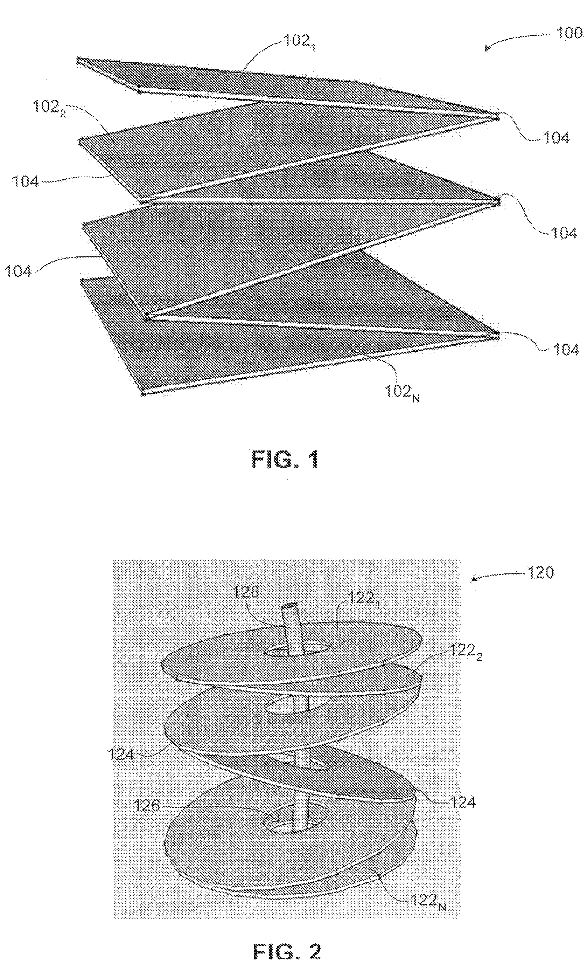

Reference is now made to Figure 1, which is a schematic illustration of a first battery configuration, generally referenced 100, constructed and operative in accordance with an embodiment of the disclosed technique. Battery configuration 100 shows how a battery can be designed such that it can be incorporated in a string-shaped implantable medical device while enabling the medical device to have a significant amount of flexibility. For example, battery configuration 100 can be achieved using thin film battery technology, and more specifically Figure 1 shows how three dimensional (herein abbreviated 3D) thin film battery technology, which can be configured to have a long and narrow flexible shape, as shown in Figure 1, can be integrated into a string-shaped implantable medical device. 3D thin film batteries are known in the art, examples of which are disclosed in the following prior art documents: U.S. Patent No. 6,197,450, U.S. Patent No. 7,527,897, U.S. Patent No. 7,618,748 and U.S. Patent Application Publication No. 2006/0032046. In addition, other micro-sized energy storage cells can be used to create the battery configuration as shown in Figure 1. For example, Reissued U.S. Patent Nos. RE41,578 and RE42,273 describe thin film micro-electrochemical energy storage cells which can be formed and coupled as shown in Figures 1 and 2 such that they can be incorporated into a string-shaped implantable medical device. As shown below, any known thin film battery can be used and configured according to the disclosed technique. For example, two dimensional (herein abbreviated 2D) battery technology can be used to construct the battery configuration shown in Figures 1 and 2.

Battery configuration 100 includes a plurality of thin film batteries 102₁, 102₂ and 102_{N}. Each thin film battery is a battery onto itself, yet can be coupled with another thin film battery via a pair of connectors (not shown), thus forming a continuous thin film battery of greater power. As shown in Figure 1, each of plurality of thin film batteries 102₁, 102₂ and 102_{N} is coupled to its neighboring thin film battery at locations 104. The connectors (not shown) at locations 104 enable each one of plurality of thin film batteries 102₁, 102₂ and 102_{N} to rotate, at least partially, around an axis (not shown), similar to a hinge. Plurality of thin film batteries 102₁, 102₂ and 102_{N} thus forms an accordion-like shape. Similar to an accordion, plurality of thin film batteries 102₁, 102₂ and 102_{N} is thus flexible due to the ability of each thin film battery to rotate around the axes of locations 104. It is noted that each one of plurality of thin film batteries 102₁, 102₂ and 102_{N} may be a rigid surface, such as a silicon substrate, or may be fabricated from a flexible material. As rigid surfaces, plurality of thin film batteries 102₁, 102₂ and 102_{N} still provide flexibility to an implantable medical device they are integrated with, since each thin film battery can partially rotate around the axis at which it is coupled with an adjacent or neighboring thin film battery. The volume taken up by battery configuration 100 can be decreased by folding each one of plurality of thin film batteries 102₁, 102₂ and 102_{N} on top of one another completely (not shown). The plurality of thin film batteries thus forms one long continuous battery. It is also noted that battery configuration 100 can be formed from a single flexible thin film battery which is folded over multiple times in an accordion-like manner. In addition, it is also possible to rebuild used regular batteries into the battery configuration shown in Figure 1, such that batteries not built from thin film technology can be used with the disclosed technique. Battery configuration 100 may also include hybrid battery chemistry in which a first portion of the thin film batteries are used for constant powering (e.g., in the case of sensing electrical activity of an organ) whereas a second portion of the thin film batteries are used for occasional powering (e.g., in the case of electric shock delivery) or for high current drain applications during limited periods.

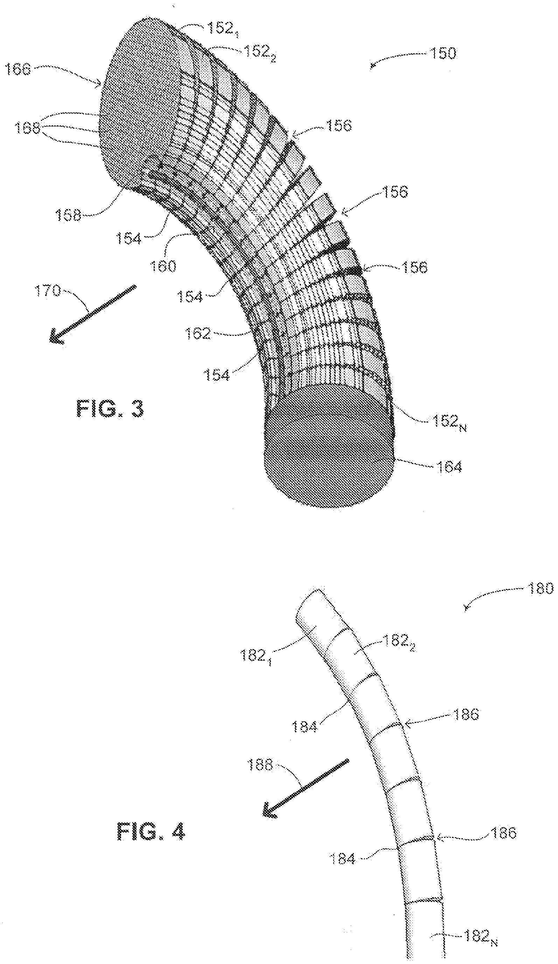

Reference is now made to Figure 2, which is a schematic illustration of a second battery configuration, generally referenced 120, constructed and operative in accordance with another embodiment of the disclosed technique. Battery configuration 120 is similar to battery configuration 100 (Figure 1), including a plurality of thin film batteries 122₁, 122₂ and 122_{N} each coupled with its neighboring thin film battery at a location 124. As mentioned above, the plurality of batteries can be other types of batteries and not just those using thin film technology. The mention of thin film technology batteries herein is merely brought as an example of how to embody the disclosed technique. The disclosed technique however applies to any kind of battery which can be formed so as to give a string-shaped implantable medical device sufficient flexibility. However, in Figure 2, each thin film battery has been reshaped as a disc. It is noted that according to the disclosed technique, the thin film batteries may be shaped into any desirable shape. The disc shape of the thin film batteries in flexible battery configuration 120 includes a central hole 126. Once each one of plurality of thin film batteries 122₁, 122₂ and 122_{N} is folded onto its neighbor, battery configuration 120 will have a cylindrical and flexible shape and can thus be inserted into a tubular or string-like structure, thus simplifying its insertion into and removal from medical devices having a string-like shape. In addition, due to central hole 126, a space or channel is created within battery configuration 120 wherein wires, cables and connections can be passed through. As shown below in Figures 3 and 6, when the battery configuration is integrated into a string-shaped implantable medical device, the channel of central hole 126 can be used to couple various parts and components of the device together. For example, as shown in Figure 2, a wire 128 can be threaded through central hole 126. In addition, the channel of central hole 126 can be used to insert wires, guidewires and stylets through, for example when the device is being initially implanted in a patient (not shown). It is noted that central hole 126 does not need to necessarily be centered in each one of plurality of thin film batteries 122₁, 122₂ and 122_{N}. The disc shape of plurality of thin film batteries 122₁, 122₂ and 122_{N} can be formed such that a hole of any shape, size and location is possible. The hole (not shown) may be, for example, square or triangular in shape. The hole (not shown) may be off-centered or located at one of the edges of each thin film battery, as shown below in Figure 3. Furthermore, the hole (not shown) may be larger or smaller in diameter than central hole 126.

Reference is now made to Figure 3, which is a schematic illustration of a third battery configuration, generally referenced 150, constructed and operative in accordance with a further embodiment of the disclosed technique. Third battery configuration 150 is similar to second battery configuration 120 (Figure 2) and includes a plurality of thin film batteries 152₁, 152₂ and 152_{N}. As mentioned above, the batteries may also be fabricated using technologies other than thin film battery technology. However, in Figure 3, as opposed to Figures 1 and 2, each thin film battery is coupled with its neighbor at points 154, where each point 154 is located on the same side of battery configuration 150. As such, battery configuration 150 can be bent in one general direction, shown by an arrow 170, thus giving battery configuration 150 a measurable amount of flexibility. As battery configuration 150 is bent in the direction of arrow 170, a plurality of spaces 156 forms between adjacent thin film batteries on the side opposite where each point 154 is located. As an example of one type of battery which can be used with the disclosed technique, a surface 166 of thin film battery 152₁ is shown, showing a plurality of holes 168 which are each filled with an electrochemical substance for storing charge and electrical energy. Such a thin film battery (although not the battery configuration as shown in Figure 3) is described in Reissued U.S. Patent No. RE41,578, as mentioned above.

In addition, unlike the battery configuration of Figure 2, each one of plurality of thin film batteries 152₁, 152₂ and 152_{N} is formed in the shape of a circle, with a small circular portion 158 cut out on the side where each point 154 is located. Thus, similar to central hole 126 (Figure 2), a channel 160 is formed by each small circular portion 158 such that a wire 162 can pass there through. As mentioned above, the formed channel does not need to be centrally located on each thin film battery. According to the disclosed technique, channel 160 can be formed anywhere on the surface of the thin film batteries, and not just in the center or on the edge of the thin film batteries; thus the examples of a channel as shown in Figures 2 and 3 are merely brought as examples. In addition, the battery configuration of the disclosed technique may include a plurality of channels formed within the thin film batteries, for example a central channel (not shown) and channel 160. As shown as well in Figure 3, an end of battery configuration 150 includes an electronics unit 164, which is coupled with wire 162. Electronics unit 164 may include a processor (not shown), at least one capacitor and other electronics necessary for the functioning of the implantable medical device battery configuration 150 is inserted into.

Reference is now made to Figure 4, which is a schematic illustration of a fourth battery configuration, generally referenced 180, constructed and operative in accordance with another embodiment of the disclosed technique. Fourth battery configuration 180 is similar to the other battery configurations disclosed thus far, however, fourth battery configuration 180 includes a plurality of segments 182₁, 182₂ and 182_{N}. Each one of plurality of segments 182₁, 182₂ and 182_{N} includes a plurality of thin film batteries. Thus segment 182₁ includes a plurality of thin film batteries (not shown), segment 182₂ also includes a plurality of thin film batteries (not shown) and segment 182_{N} further includes a plurality of thin film batteries (not shown). Alternatively, each one of plurality of segments 182₁, 182₂ and 182_{N} may include a single rigid battery. The thin film batteries in each segment are rigidly coupled with one another. As shown, each segment is coupled to an adjacent segment at points 184. Similar to Figure 3, segments are coupled together on the same side, thus forming spaces 186 when battery configuration 180 is bent in the direction of an arrow 188. Battery configuration 180 is actually semi-flexible in nature as compared to the battery configurations in Figures 2 and 3, since each segment includes a plurality of thin film batteries which are rigidly coupled with one another. Nonetheless, battery configuration 180 is flexible due to the segmentation of its parts (i.e., plurality of segments 182₁, 182₂ and 182_{N}) and enables easy insertion into and removal from a string-like medical device, whether implantable or non-implantable. Due to the constraints of a string-like medical device, especially one which is implantable, each one of plurality of segments 182₁, 182₂ and 182_{N} should be smaller than 11 millimeters in diameter and shorter than 5 centimeters in length.

Reference is now made to Figure 5, which is a schematic illustration of an implantable medical device having a flexible string shape, generally referenced 200, constructed and operative in accordance with a further embodiment of the disclosed technique. Implantable medical device 200 includes a sheath 202, having a thickness 204 and a hollow space 206. Implantable medical device 200 is designed to house various elements such as a sensing electrode (not shown), a signal delivery electrode (not shown) and possibly also electronics (not shown) along sheath 202 as shown by an arrow 208. Such an implantable medical is described in U.S. Provisional Patent Application No. 61/728,897 and U.S. Provisional Patent Application No. 61/765,195, as mentioned above. As described below, hollow space 206 can be used to house a battery having one of the battery configuration described above in Figures 1-4. Hollow space 206 substantially houses a core, which includes one of the battery configurations described above along with addition electronics required for providing electrical impulses and stimulation therapies. It is noted that the battery configurations described above may employ hybrid battery chemistry in which the components comprising the battery configuration are sub-divided into a plurality of groups or portions. For example, a first portion of the components comprising the battery configuration might be used to constantly power parts of implantable medical device 200 which require a constant source of power, such as a processor and electronics (both not shown) for recording the sensed electrical activity of an internal organ and determining what kind of electrical impulse should be delivered to the internal organ. A second portion of the components comprising the battery configuration might be used to occasionally power parts of implantable medical device 200 which are only used in certain circumstances. For example, if implantable medical device 200 includes at least one capacitor for storing the energy required for generating a high voltage shock and voltage amplification electronics, then the aforementioned second portion may be used for powering the amplification electronics used for building up voltage on the at least one capacitor. In general, one of the main type of therapies that a medical device, such as an ICD, can deliver is the application of electric shocks to organs or tissues in the body. At least one capacitor is used to store electrical energy required for generating a high voltage. An electric shock to be delivered as the therapy is the discharging of the stored electrical energy through the organs or tissues of a patient. Since high voltage shocks are not administered constantly but only under certain circumstances, the life of a battery having one of the configurations mentioned above can be extended by dedicating a portion of the battery to being constantly used whereas another portion of the battery is used only when needed. In another embodiment, a portion of the battery is used for high current intermittent applications whereas the other portion is used for lower current continuous applications. Hybrid chemistry to support these dual functions can also be incorporated into such a battery.

Reference is now made to Figure 6, which is a schematic illustration of a battery integrated into the implantable medical device of Figure 5, generally referenced 220, constructed and operative in accordance with another embodiment of the disclosed technique. As shown, an implantable medical device 222 includes a sheath 230 and a hollow space 228. A battery 224 is positioned in hollow space 228 in sheath 230. Battery 224 has a configuration according to the disclosed technique, such as those described above in Figures 1-4 and can be referred to as a core structure. Also as shown, implantable medical device 222 has a flexible string shape. Figure 6 shows how according to the disclosed technique, battery 224 can be replaced easily and simply without having to remove implantable medical device 222 from a patient (not shown), thus not requiring any major surgery to replace battery 224. Implantable medical device 222 is positioned subcutaneously in a patient. It is noted that implantable medical device 222 may also be positioned in a patient such that a first part of the device is positioned subcutaneously whereas a second part of the device is positioned internally, such as under the ribs. Implantable medical device 222 may include a cover (not shown) at each end for enclosing battery 224 within hollow space 228. The cover may also serve the purpose of electrically coupling battery 224 to any electronics (not shown) housed in sheath 230, such as found in certain handheld flashlight designs. As mentioned in Figure 5, sheath 230 may house various electrodes (not shown) as well as other elements of the implantable medical device (excluding the battery) which do not typically require replacement over time, such as electronics and capacitors (not shown). As mentioned above, battery 224 is electrically coupled (not shown) with sheath 230 and any electronics housed therein. According to another embodiment of the disclosed technique, such as the battery configuration shown above in Figure 3, any electronics and capacitors may be coupled with battery 224 and configured to also fit within hollow space 228. In such an embodiment, battery 224 is coupled with sheath 230 such that the electrodes in sheath 230 are coupled with the electronics coupled with battery 224. In a further embodiment, battery 224 may be constructed to include electronics (not shown) and at least one capacitor (not shown), thus also forming a core structure. In all embodiments, the cover is designed to securely keep battery 224, the core structure, coupled with sheath 230 such that battery 224 is not unintentionally disconnected from sheath 230.

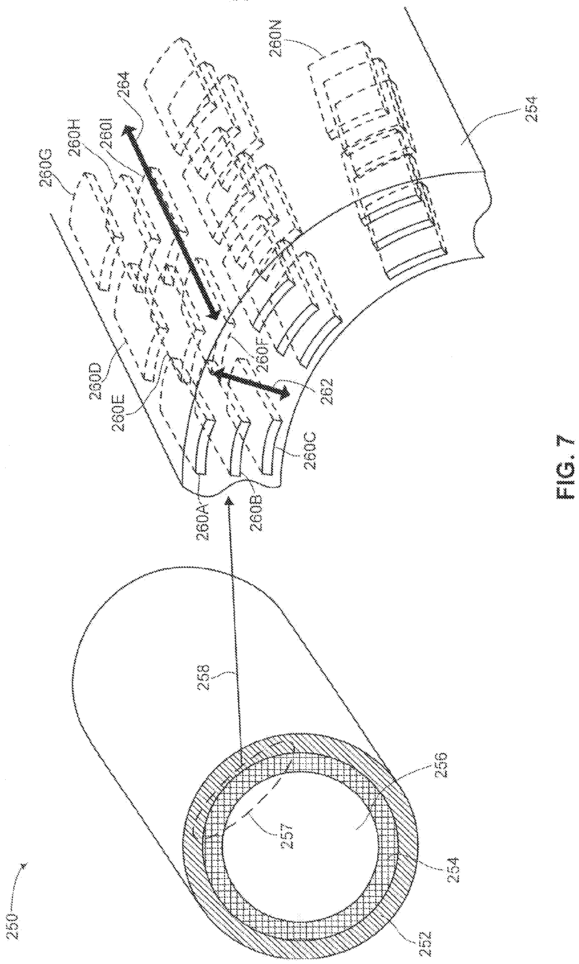

Once battery 224 is out of power and needs to be replaced, battery 224 can be easily pulled out of sheath 230, as shown by an arrow 226, through a small incision (not shown) made in the skin of a patient just above the position of the cover. Once the old battery is pulled out, a new battery or core structure (not shown) can then be inserted into hollow space 228 through the incision. The incision can then be easily sewed up. Thus sheath 230 can be left in a patient and does not need to be removed in order to replace the battery of the implantable medical device. The replacement of battery 224 can thus be performed easily and quickly without causing any unnecessary pain or discomfort to the patient. When implantable medical device 222 is implanted subcutaneously, battery 224 can be easily replaced via a minor surgical procedure. A small incision is made in the area where a proximal end 232 of implantable medical device 222 is located. Proximal end 232 is then exposed and a cover (not shown) covering proximal end 232 is opened and temporarily removed. Battery 224 inside sheath 230 is pulled out and a new fresh battery (not shown) is inserted. The new battery may need to be coupled electrically with sheath 230 or may be coupled electrically once the cover is put back. The cover is then put back on proximal end 232 and the small incision is sutured. As described, battery 224 (which is a core structure) can be replaced without having to remove sheath 230 from the patient, thereby greatly simplifying the procedure by which the power source of an implantable medical device is replaced. Such a procedure is fast and easy and does not require any major surgery. In general, since implantable medical device 222 will have been inside a patient for quite a bit of time before battery 224 needs to be replaced, during that time sheath 230 will have become coupled with the tissue surrounding it, therefore as battery 224 is removed, sheath 230 will remain in place. Thus battery 224 can be replaced without having to remove sheath 230 from the patient. It is noted as well that implantable medical device 222 has a rigid outer shape (i.e., sheath 230), such that when battery 224 is removed, sheath 230 retains its shape so that a new battery can be inserted into hollow space 228 without too much difficulty. As described below in Figure 7, the sheath surrounding the battery may also surround the electronic components of the implantable medical device. In such an embodiment (not shown in Figure 6), the sheath enables the electronic components, which may be formed in a string-like shape, to be easily removed and inserted. Therefore, just as battery 224 can be easily removed and reinserted into sheath 230, the electronic components can also be easily removed and reinserted into sheath 230.

Reference is now made to Figure 7, which is a schematic illustration and close-up of an implantable medical device with a removable battery, generally referenced 250, constructed and operative in accordance with a further embodiment of the disclosed technique. Implantable medical device 250 includes an outer sheath 252, a battery 254 and a main unit 256. Outer sheath 252, battery 254 and main unit 256 each have a generally circular cross-sectional shape, thus enabling main unit 256 to be inserted into battery 254 and battery 254 to be inserted into outer sheath 252. Each one of outer sheath 252, battery 254 and main unit 256 has a flexible shape. Outer sheath 252 may be constructed from a semi-rigid material, thus enabling battery 254 and main unit 256 to be removed from outer sheath 252 with outer sheath 252 maintaining its shape. Battery 254 and main unit 256 are both core structures which can be easily removed and inserted into outer sheath 252. In general, battery 254 and main unit 256 can be constructed as a single core structure or as two separate core structures. In this manner, with implantable medical device 250 implanted subcutaneously in a patient, battery 254 and main unit 256 can be simply removed from outer sheath 252 while leaving outer sheath 252 still inside the patient. According to the disclosed technique, a new battery, a new main unit or both can then be easily reinserted into outer sheath 252.

Main unit 256 may include all the elements and components needed for implantable medical device 250 to function minus its power source. For example, main unit 256 may include electrical leads (not shown), capacitors (not shown), a processor (not shown) and other necessary electronics for providing electrical impulses to the patient. Power is provided to these elements and components from battery 254. As mentioned above, battery 254 may include hybrid battery chemistry, where a portion of battery 254 is used to constantly power elements like a processor or provide electrical impulses to electrical leads and another portion of battery 254 may be used to occasionally load the capacitors when needed. In one embodiment, as shown, main unit 256 and battery 254 are two separate entities which are coupled together. In another embodiment (not shown), main unit 256 and battery 254 are constructed as a single entity. A section 257 of implantable medical device 250 is shown in a close-up, as indicated by an arrow 258. The close-up is of battery 254 and how it is constructed.

Battery 254 has a generally circular cross-sectional shape, having a particular thickness, as shown by an arrow 262 and a particular length, as shown by an arrow 264. Battery 254 includes a plurality of small 3D thin film batteries 260A, 260B, 260C, 260D, 260E, 260F, 260G, 260H, 260I and 260N. The plurality of 3D thin film batteries can be arranged in rows and columns along the length and thickness of battery 254. As shown in Figure 7, 3D thin film batteries 260A, 260B and 260C are arranged in a column and 3D thin film batteries 260A, 260D and 260G are arranged in a row. The plurality of 3D thin films batteries shown is merely schematic. Battery 254 may include thousands of small 3D thin film batteries arranged in a compact configuration within the thickness of battery 254. Each one of 3D thin film batteries 260A-260N may be flexible or rigid. In either case, their configuration within the thickness of battery 254 provides battery 254 with a degree of flexibility. As shown in Figure 7, battery 254 substantially represents a plurality of layers of small 3D thin film batteries surrounding and encompassing main unit 256. Battery 254 is coupled with main unit 256 such that battery 254 can provide power to the various components of main unit 256. Various methods of coupling a battery to components which require power are known in the art. The type of coupling of battery 254 to main unit 256 is thus a matter of design choice and is known to the worker skilled in the art.

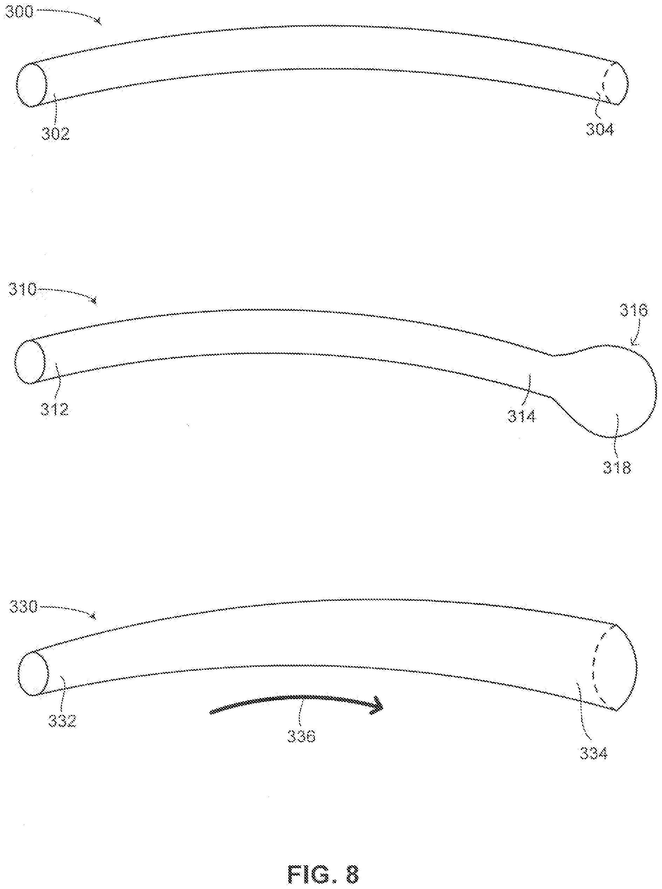

Reference is now made to Figure 8 which is a schematic illustration of various possible shapes for an implantable medical device having a flexible string shape, generally referenced 300, 310 and 330 respectively, constructed and operative in accordance with another embodiment of the disclosed technique. Flexible string shapes 300, 310 and 330 may be the shape of a core structure including a battery and other electronics, such as battery 254 (Figure 7), main unit 256 (Figure 7) or both, as described above. Flexible string shapes 300, 310 and 330 may also be the shape of a sheath into which a core structure including a battery and other electronics may in inserted into, such as outer sheath 252 (Figure 7), as described above. In general, each of the flexible string shapes described below is described as having a proximal end and a distal end. These labels however are merely for the purposes of describing the shapes and can easily be switched, such that the proximal end is referred to as the distal end and the distal end is referred to as the proximal end. Flexible string shape 300 includes a proximal end 302 and a distal end 304. Flexible string shape 300 has a generally cylindrical or tubular shape, characterized by a generally uniform cross-sectional shape and diameter along its length. Flexible string shape 310 includes a proximal end 312 and a distal end 316. Distal end 316 includes two sections, a bulbous end section 318 and an adjacent end structure 314. From proximal end 312 to adjacent end structure 314, flexible string shape 310 substantially resembles flexible string shape 300, having a generally cylindrical or tubular shape, characterized by a generally uniform cross-sectional shape and diameter along its length. However, distal end 316 has bulbous end section 318 which is larger in diameter than adjacent end structure 314. Bulbous end section 318 has a generally spherical or ellipsoidal shape, giving flexible string shape 310 on the whole a shape which resembles a tadpole. Flexible string shape 310 is one continuous shape, having bulbous end section 318 at its distal end. Bulbous end section 318 can be used to house a component of a medical device which cannot fit inside the section of flexible string shape 310 from proximal end 312 to adjacent end structure 314. For example, if at least one capacitor (not shown) is to be included in a medical device embodied as having a string shape, and the at least one capacitor is too large to be encapsulated along the length of the flexible string shape in its diameter then the at least one capacitor may be placed in bulbous end section 318. Additional electronic components may also be placed in bulbous end section 318 for coupling a plurality of capacitors together in order to generate a desired high voltage and specific waveform for a given stimulation therapy to be administered by the medical device, whether implantable or not. It is noted that if flexible string shape 310 is embodied as an implantable medical device then when implanted in a patient, proximal end 312 may be the distal end first inserted into the patient and distal end 316 may be the proximal end located near an incision made into the patient to insert the implantable medical device. Flexible string shape 330 includes a proximal end 332 and a distal end 334. Unlike flexible string shapes 300 and 310, flexible string shape 330 has a generally truncated conoid shape along its length. As shown in Figure 8, in the direction of an arrow 336, the cross-section of the generally tubular or cylindrical shape of flexible string shape 330 changes over length, with the diameter of a cross-section of proximal end 332 increasing in the direction of distal end 334. Similar to flexible string shape 310, the increase in diameter over length of flexible string shape 330 enables larger components to be inserted into a medical device having such a shape. Therefore, a capacitor or other large electronic component (both not shown) which would not fit in proximal end 332 may be inserted in distal end 334 which has a larger diameter in its cross-section.

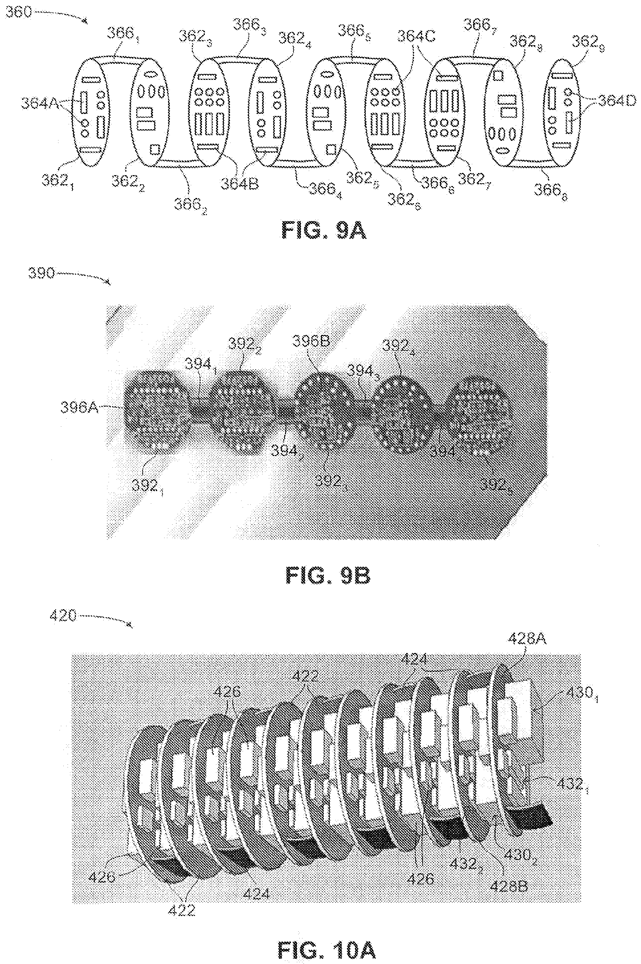

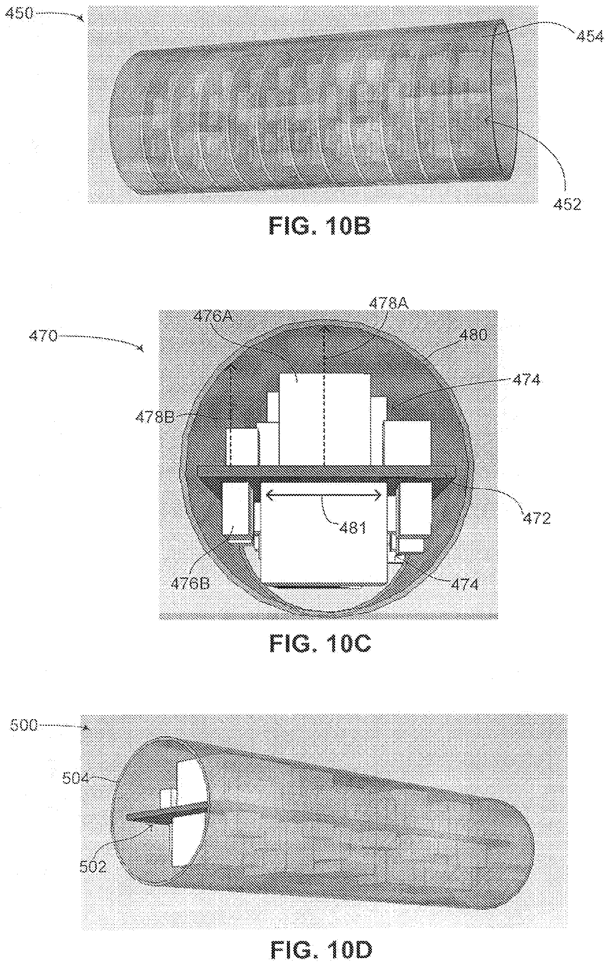

In a similar manner to third battery configuration 150 (Figure 3), electronic components to be used in a flexible medical device can be shaped and configured to fold in a cylindrical manner such that they can be encapsulated in a string-like or snake shaped medical device, whether implanted or not. Various embodiments for configuring electronic components in such a structure are described below in Figures 9A-10F. Reference is now made to Figure 9A, which is a schematic illustration of an encapsulation configuration for electronic components in a flexible implantable medical device, shown in an unfolded view, generally referenced 360, constructed and operative in accordance with a further embodiment of the disclosed technique. Folding the electronic components in such manner enables them to later on be encapsulated into a cylindrical shape envelope or encasing and to become a part of a flexible string shape or snake-like shape medical device. As shown in Figure 9A, electronic components in a medical device can be placed and divided up amongst a plurality of cylindrically or circularly shaped circuit boards (herein referred to as CB) 362₁-362₉. Each one of CBs 362₁-362₉ includes a plurality of electronic components, such as capacitors, resistors, transistors, switches, processors, transformers, diodes, ASICs (application specific integrated circuit), FPGAs (field-programmable gate arrays) and the like. For example, CB 362₁ includes plurality of electronic components 364A, CBs 362₃ and 362₄ include plurality of electronic components 364B, CBs 362₆ and 362₇ include plurality of electronic components 364C and CB 362₉ includes plurality of electronic components 364D. Each one of CBs 362₁-362₉ has a substantially similar cylindrical or circular shape such that each CB can be placed adjacent to a subsequent CB. CBs 362₁-362₉ are electrically coupled sequentially using a plurality of flat connection cables 366₁-366₈. Flat connection cable 366₁ couples CB 362₁ with CB 362₂, flat connection cable 366₂ couples CB 362₂ with CB 362₃, flat connection cable 366₃ couples CB 362₃ with CB 362₄, flat connection cable 366₄ couples CB 362₄ with CB 362₅, flat connection cable 366₅ couples CB 362₅ with CB 362₆, flat connection cable 366₆ couples CB 362₆ with CB 362₇, flat connection cable 366₇ couples CB 362₇ with CB 362₈ and flat connection cable 366s couples CB 362s with CB 362₉. As shown, plurality of flat connection cables 366₁-366₈ electrically couple between CBs alternatively at opposite ends of each CB, such as either the top of a CB or the bottom of a CB. Plurality of flat connection cables 366₁-366₈ are flexible and are long enough to enable a first CB to be folded directly over a subsequent CB. Plurality of flat connection cables 366₁-366₈ can also be embodied as connection cables which are not flat. In addition, other methods for coupling adjacent CBs together can be used in the disclosed technique, such as via male-female connector pairs positioned on opposite sides of adjacent CBs. Flat connection cables 366₁, 366₃, 366₅ and 366₇ electrically couple CBs at the top (i.e., at one side) of a CB whereas flat connection cables 366₂, 366₄, 366₆ and 366₈ electrically couple CBs at the bottom (i.e., at an opposite side) of a CB. This alternative coupling, as shown below in Figures 10A and 10B, enables one CB to be folded on top of another CB in a pleated manner, thereby forming a cylindrically shaped electronics configuration which can be encapsulated in a cylindrical enclosure.

Reference is now made to Figure 9B, which is an image of electronic components in the encapsulation configuration of Figure 9A, generally referenced 390, constructed and operative in accordance with another embodiment of the disclosed technique. Shown in Figure 9B is a plurality of CBs 392₁-392₅ shaped in a circular fashion, electrically coupled sequentially by a plurality of flat connection cables 394₁-394₄. Plurality of flat connection cables 394₁-394₄ are each flexible and long enough to enable one CB to be folded onto its neighboring CB. Each CB includes a plurality of electronic components, such as electronic components 396A on CB 392₁ and electronic components 396B on CB 392₃. Plurality of CBs 392₁-392₅ can be folded one on top of the other in a pleated or accordion-like manner, thereby forming a compact cylinder. As is understood by the worker skilled in the art, once folded, plurality of flat connection cables 394₁-394₄ indeed coupled sequential CBs alternatively at their tops and bottoms.

Reference is now made to Figures 10A and 10B, which are schematic illustrations of the encapsulation configuration of Figures 9A and 9B shown in a folded view, generally referenced 420 and 450 respectively, constructed and operative in accordance with a further embodiment of the disclosed technique. With reference to Figure 10A, a plurality of circular shaped CBs 422 are shown folded in an accordion-like or pleated manner. Sequential CBs are alternatively electrically coupled at the top and bottom (i.e., at opposite sides) of each CB with a flexible flat connection cable, shown as a plurality of flexible flat connection cables 424. Each flexible flat connection cable is long enough such that adjacent CBs can be folded one on top of the other with the flexible flat connection cable still having enough slack such that no mechanical stress is placed upon the flexible flat connection cable. Each one of plurality of circular shaped CBs 422 includes a plurality of electronic components 426.

According to the disclosed technique, optimal volume consumption of the configuration of electronic components shown is achieved by placing plurality of electronic components 426 on both sides of a CB. Optimal volume consumption relates to minimizing the amount of volume an electronic components configuration occupies. As shown, a circular shaped CB 428A includes electronic components on both sides. Optimal volume consumption is also achieved by the specific positioning of electronic components on a CB based on the dimensions (for example height) of each electronic component. For example, a CB 428A includes a relatively tall electronic component 430₁ and a relatively short electronic component 432₁ on one side and a relatively tall electronic component 430₂ on its other side. A CB 428B also includes relatively tall electronic components (not labeled) and a relatively short electronic component 432₂. Relatively tall electronic component 430₂ is positioned on CB 428A such that when CB 428A is folded onto CB 428B, relatively tall electronic component 430₂ from CB 428A will sit directly over relatively short electronic component 432₂ from CB 428B. In this respect, electronic components on each CB are positioned based on their height such that when adjacent CBs are folded on top of one another, volume consumption is maximized by complementarily placing relatively tall electronic components over relatively short electronic components and vice-versa. As seen in Figure 10A, when electronic components are positioned according to the disclosed technique, plurality of circular shaped CBs 422 can be folded up into a cylindrical shape while minimizing the volume required to encase the electronic components of each CB.

With reference to Figure 10B, the encapsulation configuration of electronic components of Figure 10A is shown, delineated by an arrow 452. Encapsulation configuration of electronic components 452 can now be encased in a protective cylinder 454. Protective cylinder 454 may be made from a metal, such as titanium, or from a plastic material. Protective cylinder has a relatively small diameter, and can have for example an inner diameter of 11 millimeters (herein referred to as mm). Encapsulation configuration of electronic components 452 may represent an electronics unit within an implantable medical device and may have a diameter which is smaller than 11 millimeters and a length which is less than 5 centimeters.

Reference is now made to Figures 10C and 10D, which are schematic illustrations of another encapsulation configuration for electronic components in a flexible implantable medical device, generally referenced 470 and 500 respectively, constructed and operative in accordance with another embodiment of the disclosed technique. With reference to Figure 10C, a cross-sectional view of another encapsulation configuration for electronic components is shown. In this configuration, a single flat CB 472 is used to couple electronic components together. Flat CB 472 has a generally rectangular shape, being long and narrow. Flat CB 472 includes a plurality of electronic components 474, positioned on both sides of flat CB 472.

In Figure 10C, optimal volume consumption of plurality of electronic components 474 is achieved by positioning taller electronic components, such as electronic component 476A along a center line (not shown) of flat CB 472, whereas shorter electronic components, such as electronic component 476B are positioned closer to the edges (not labeled) of flat CB 472. Flat CB 472 and plurality of electronic components 474 are encased in a protective cylinder 480. Protective cylinder 480 may be made from a metal, such as titanium, having an inner diameter of 11 millimeters (herein referred to as mm). As shown, the vertical distance between flat CB 472 and protective cylinder 480 various along a width 481 of flat CB 472. In the center (not labeled) of flat CB 472, the vertical distance is at a maximum, as shown by a dashed arrow 478A. As the edges of flat CB 472 are approached, the vertical distance approaches a minimum, as shown by a dashed arrow 478B. As understood by the worker skilled in the art, appropriate placing of the electronic components on flat CB 472 as described above can optimize the volume consumption of the electronic components in protective cylinder 480.

With reference to Figure 10D, a perspective view of the encapsulation configuration for electronic components of Figure 10C is shown. Figure 10D includes a flat CB with a plurality of electronic components 502, as described above in Figure 10C. Flat CB with plurality of electronic components 502 is encased in a protective cylinder 504. As seen optimal volume consumption by flat CB with plurality of electronic components 502 is achieved in protective cylinder 504 according to the disclosed technique.

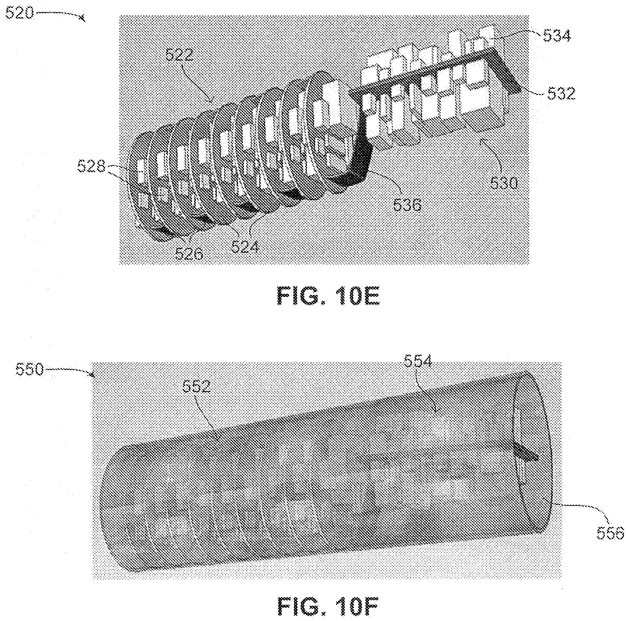

Reference is now made to Figures 10E and 10F, which are schematic illustrations of a further encapsulation configuration for electronic components in a flexible implantable medical device, generally referenced 520 and 550 respectively, constructed and operative in accordance with a further embodiment of the disclosed technique. With reference to Figure 10E, encapsulation configuration for electronic components 520 is shown which substantially is a hybrid between the encapsulation configurations shown in Figures 10A-10B and 10C-10D. Encapsulation configuration for electronic components 520 includes a first section 522 wherein a plurality of flat CBs 524 are electrically coupled together sequentially at opposite ends of adjacent CBs by a plurality of flexible flat connection cables 526. A plurality of electronic components 528 are positioned on both sides of each of plurality of flat CBs 524 to achieve optimal volume consumption, as described above in Figures 10A and 10B. Encapsulation configuration for electronic components 520 also includes a second section 530 wherein a single rectangular shaped flat CB 532 includes a plurality of electronic components 534, positioned on both sides of flat CB 532 to achieve optimal volume consumption, as described above in Figures 10C and 10D. A longer flexible connection cable 536 electrically couples section 522 with section 530.

With reference to Figure 10F, encapsulation configuration for electronic components 550 is shown including a first section 552 configured like section 522 (Figure 10E) and a second section 554 configured like section 530 (Figure 10E). Both first section 552 and second section 554 can be encased in a protective cylinder 556, thereby maximizing volume consumption of the electronic components in protective cylinder 556. As electronic components come in a variety of shapes and sizes, the advantage of the encapsulation configurations shown in Figures 10E and 10F is that generally smaller electronic components in a medical device can be positioned according to the configuration shown in the first sections (like in Figures 10A and 10B), where more electronic components may be positioned in a given volume, whereas generally larger electronic components in the medical device can be positioned according to the configuration shown in the second sections (like in Figures 10C and 10D), which affords more volume especially for tall electronic components.

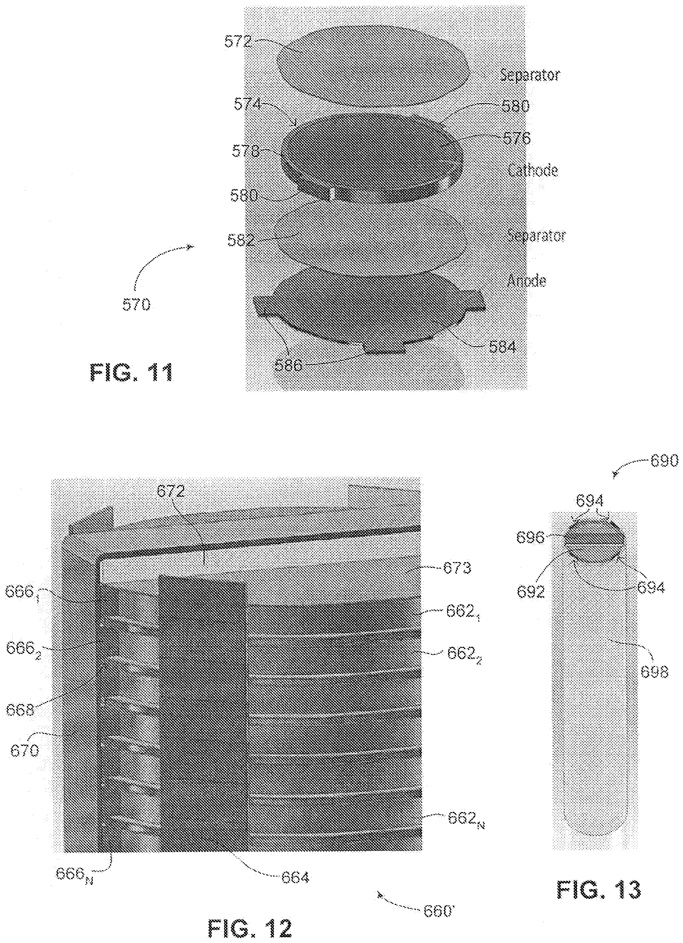

Reference is now made to Figure 11, which is a schematic illustration of a single flat battery chip, shown in an exploded view, generally referenced 570, constructed and operative in accordance with another embodiment of the disclosed technique. Figure 11 shows a single flat battery chip 570 in an exploded view. Single flat battery chip is not a functional battery but includes the necessary parts for building a battery. Single flat battery chip 570 includes a cathode 574 and an anode 584. The eventual battery chemistry as described below in Figure 13 is able to produce high power and high current to enable charging a capacitor to around 1250 volts and around 70 joules of energy in under 12 seconds. In some embodiments of the disclosed technique, single flat battery chip 570 may be embodied as a three-dimensional thin film battery (herein referred to as 3D-TFB) or a semi-3D-TFB, as disclosed in U.S. Patent Nos. 6,197,450, 7,527,897, 7,618,748, reissued U.S. Patent Nos. RE41,578 and RE42,073, and U.S. Patent Application Serial No. 13/988,337. Single flat battery chip 570 can be combined with other single flat battery chips (not shown) as shown below in Figure 12. Cathode 574 is covered by a first separator 572, while cathode 574 and anode 584 are separated by a second separator 582. First separator 572, cathode 574, second separator 582 and anode 584 are substantially circular in shape. Cathode 574 and anode 584 are made from known materials used for constructing cathodes and anodes. First separator 572 and second separator 582 are made from partially electrically insulating materials, such as porous polymers. Anode 584 includes four anode extensions 586. Anode 584 may include at least one anode current collector (not shown). Anode extensions 586 may be positioned anywhere along the circumference of anode 584. For example, anode extensions 586 are positioned approximately 90 degrees from one another. Anode 584 and anode extensions 586 may be coated with a current collector material, such as copper foil. Anode extensions 586 may have a thickness of approximately 20 microns. Cathode 574 includes a body 578, two cathode extensions 580 and an active cathode material 576. Cathode extensions 580 aid in cathode current collection, as described below. Active cathode material 576 may be incorporated into body 578 in a semi-3D-TFB or 3D-TFB configuration, as mentioned above. Active cathode material 576 may extend over to cathode extensions 580. Cathode 574 can act as its own current collector if conductive enough. Alternatively, cathode extensions 580 may serve as a cathode current collector as described below. Cathode 574 can include at least one cathode extension (not shown). The number of cathode and anode extensions can be equal (not shown) or unequal (as shown in Figure 11A). Cathode extensions 580 may be positioned anywhere along the circumference of cathode 574. For example, cathode extensions 580 are positioned approximately 180 degrees from one another. Cathode extensions 580 and anode extensions 586 are positioned such that they do not overlap one another. Body 578 can be made from a hard material such as silicon or glass, for example in the form of a perforated silicon substrate or a glass capillary array (herein referred to as GCA). Active cathode material 576 may be made from any known cathode material, such as gold or the materials used in lithium ion batteries or lithium TFBs (see for example U.S. Patent Nos. 6,197,450, 7,527,897, 7,618,748, reissued U.S. Patent Nos. RE41,578 and RE42,073, and U.S. Patent Application Serial No. 13/988,337. In another embodiment of the disclosed technique, body 578 may be made of a soft or flexible material such as a polymer, a plastic or rubber. Cathode extensions 580 are substantially thicker than anode extensions 586, and may be as thick as 500-1000 microns. Active cathode material 576 is deposited on body 578, which can be embodied as a perforated disc. First separator 572 and second separator 582 may be made from a porous polymer.

Reference is now made to Figure 12, which is a schematic illustration of a plurality of single flat battery chips of Figure 11, showing how the cathodes and anodes of each single flat battery chip are coupled together, constructed and operative in accordance with a further embodiment of the disclosed technique. As shown, a plurality of single flat battery chips 662₁, 662₂ and 662_{N} are assembled together. All the anodes (not labeled) of each one of plurality of single flat battery chips 662₁, 662₂ and 662_{N} are coupled together as shown by an arrow 664, to collect electrical current from the anodes. A plurality of cathode extensions 666₁, 666₂ and 666_{N} are shown all lined up in parallel to one another. Plurality of cathode extensions 666₁-666_{N} may be covered with a cathode current collector material. Due to the presence of separators (not labeled) between each cathode and anode of each single flat battery chip and the particular design of each cathode extension, a space exists between adjacent cathode extensions, such as a gap 668. In addition, the design of each cathode extension does not touch an adjacent anode (not labeled). As shown, plurality of cathode extensions 666₁, 666₂ and 666_{N} are not electrically coupled to one another. In one embodiment, outside surfaces 661 of each cathode extension are covered with an electrically conductive coating, such as gold or nickel, so that each cathode extension is electrically coupled with the active cathode material of each single flat battery cell. In another embodiment, plurality of cathode extensions 666₁-666_{N} are made from a conductive material and thus the plurality of cathode extensions are actually a plurality of cathode current collectors. In this embodiment, as in the previous embodiment, each cathode current collector is not electrically coupled with its neighboring cathode current collector.

As shown, each one of cathode extensions 666₁-666_{N} can be coupled together using a collector band 670. Collector band 670 is made from a thin conductive metal which is substantially the width of a cathode extension. Collector band 670 wraps around the battery unit coupling cathode extensions on both sides of a single flat battery chip, thus enabling electrical current to flow from all the cathodes. Collector band 670 runs along the sides and top of the plurality of single flat battery chips. At the top of the plurality of single flat battery chips, an insulating rod 672 is placed on top of a first separator 673 of single flat battery chip 662₁ to prevent collector band 670 from making electrical contact with the anode (not labeled) of single flat battery chip 662₁. Collector band 670 thus substantially couples all the cathode extensions on each side of the plurality of single flat battery chips. As shown, all cathodes and anodes of the battery unit are coupled together, with all cathodes being electrically coupled via collector band 670 and all anodes being electrically coupled by four columns of anode extensions which touch one another. Thus each single flat battery chip is electrically coupled with its neighboring single flat battery chip in parallel. It is noted that the above description is based on the single flat battery chip of Figure 11 in which the anode of a single flat battery chip is located underneath the cathode. The disclosed technique can also be embodied with the position of the cathode and anode in a single flat battery chip reversed, in other words, with the cathode of a single flat battery chip being located underneath the anode. This embodiment is not shown in the figures but the battery unit of the disclosed technique can be embodied as such. The structure of the plurality of single flat battery chips enable a plurality of battery chips to be coupled such that the anodes and the cathodes of each battery chip are respectively coupled together. As mentioned above, the structure shown in Figure 12 is not a battery yet as it is lacking an electrolyte to enable current to flow through.

Reference is now made to Figure 13, which is a schematic illustration of the plurality of single flat battery chips of Figure 12 fully assembled into a battery, generally referenced 690, constructed and operative in accordance with another embodiment of the disclosed technique. Battery 690, once fully assembled as a plurality of single flat battery chips (not shown) is covered with a thin insulating sleeve 698, constructed from an electrically insulating material, such as non-conductive plastic. Thin insulating sleeve 698 may be rigid and may be used for assembling each single flat battery chip into battery 690. For example, the diameter of thin insulating sleeve 698 may be designed to securely hold each single flat battery chip in place while a second single flat battery chip is loaded into thin insulating sleeve 698, thus also preventing electrical shorts between single flat battery chips by preventing them from accidentally touching one another while being loaded into thin insulating sleeve 698. The diameter of thin insulating sleeve 698 may be less than the diameter of an anode (not shown) with its anode extensions (not shown) not folded such that placement of an anode inside thin insulating sleeve 698 causes the anode extensions to fold up sufficiently as shown in Figure 12 to couple adjacent anode extensions to each other. Thin insulating sleeve 698 may also include a plurality of grooves (not shown) for lining up cathode extensions and anode extensions such that as single flat battery chips are loaded into thin insulating sleeve 698, cathode extensions and anode extensions form parallel columns, as shown in Figure 12. As shown, only the top separator of the top single flat battery chip, a separator 692, is visible once thin insulating sleeve 698 has been loaded up with a plurality of single flat battery chips. Also visible are a plurality of tops 694 of the four anode extension columns (not shown) and a top part 696 of a collector band (not labeled) coupling the two cathode extension columns (not shown). Battery 690 may be placed inside a cylinder encasement (not shown). Electrical connections (not shown) can be made between plurality of tops 694 and top part 696 of the anode extensions and cathode extensions, therefore forming "plus" and "minus" terminals for battery 690. The cylinder encasement is then filled with an electrolyte (not shown) and fully sealed, thus constructing a fully functional battery (not shown). Battery 690 enables a relatively small sized battery of high power to be constructed. Unlike standard high power batteries which may include a plurality of lower power batteries coupled together, the embodiment shown in Figure 13 enables high power generation in a single battery unit which includes a plurality of flat battery chips. This is possible due to the cathode and anode extensions of each flat battery chip and their respective configurations which enable adjacent cathodes and anodes to be electrically coupled with one another without each flat battery chip forming an individual battery or battery unit.

It will be appreciated by persons skilled in the art that the disclosed technique is not limited to what has been particularly shown and described hereinabove. Rather the scope of the disclosed technique is defined only by the claims, which follow.

## Claims

1. An encapsulation configuration (420) for electronic components in a flexible implantable medical device, comprising:
a plurality of circuit boards (422), each one of said plurality of circuit boards comprising at least one electronics component (426); and
a plurality of flat connection cables (424),
**characterized in that**:
each one of said plurality of circuit boards has a generally circular shape;
each one of said plurality of flat connection cables electrically couples adjacent ones of said plurality of circuit boards alternatively at opposite ends; and
each one of said plurality of circuit boards is folded over an adjacent one of said plurality of circuit boards in a pleated manner, thereby giving said encapsulation configuration an accordion-like shape.

2. The encapsulation configuration according to claim 1, wherein each one of said at least one electronics component is specifically positioned on a respective one of said plurality of circuit boards according to its height, thereby achieving optimal volume consumption in said plurality of circuit boards.

3. The encapsulation configuration according to claim 1, wherein a relatively tall at least one electronics component (430₂) of a first one of said plurality of circuit boards (428A) is complementarily placed over a relatively short at least one electronics component (432₂) of a second one of said plurality of circuit boards (428B) and vice-versa when said first one of said plurality of circuit boards is folded over said second one of said plurality of circuit boards, thereby achieving optimal volume consumption in said plurality of circuit boards.

4. An encapsulation configuration (520) for electronic components in a flexible implantable medical device according to claim 1, further comprising:
a flat circuit board (532), comprising a plurality of electronics components (534),
wherein said flat circuit board has a generally rectangular shape;
wherein said plurality of electronics components are positioned on both sides of said flat circuit board;
wherein taller ones of said plurality of electronics components are positioned closer to the center of said flat circuit board; and
wherein shorter ones of said plurality of electronics components are positioned closer to the edges of said flat circuit board, thereby achieving optimal volume consumption in said flat circuit board,
wherein a first one of said plurality of circuit boards (524) is coupled with said flat circuit board (532) with a connection cable (536), and
wherein said encapsulation configuration has a cylindrical shape.

5. The encapsulation configuration according to claim 4, wherein said plurality of circuit boards and said flat circuit board each have a diameter equal to or smaller than 11 millimeters.

6. The encapsulation configuration according to claim 4, wherein said plurality of circuit boards and said flat circuit board together have a total length equal to or shorter than 5 centimeters.

## Patentansprüche

1. Verkapselungskonfiguration (420) für Elektronikkomponenten in einer flexiblen implantierbaren medizinischen Vorrichtung, umfassend:
mehrere Leiterplatten (422), wobei jede der mehreren Leiterplatten mindestens eine Elektronikkomponente (426) umfasst; und
mehrere Flachverbindungskabel (424),
**gekennzeichnet dadurch, dass**:
jede der mehreren Leiterplatten eine im allgemeinen kreisförmige Form aufweist;
jedes der mehreren Flachverbindungskabel benachbarte der mehreren Leiterplatten abwechselnd an gegenüberliegenden Enden elektrisch koppelt; und
jede der mehreren Leiterplatten plissiert über eine benachbarte der mehreren Leiterplatten gefaltet wird, wodurch die Verkapselungskonfiguration eine akkordeonartige Form erhält.

2. Verkapselungskonfiguration nach Anspruch 1, wobei jede der mindestens einen Elektronikkomponente entsprechend ihrer Höhe spezifisch auf einer entsprechenden der mehreren Leiterplatten positioniert ist, um so einen optimalen Volumenverbrauch in den mehreren Leiterplatten zu erreichen.

3. Verkapselungskonfiguration nach Anspruch 1, wobei eine relativ hohe mindestens eine Elektronikkomponente (430₂) einer ersten der mehreren Leiterplatten (428A) komplementär über einer relativ kurzen mindestens einen Elektronikkomponente (432₂) einer zweiten der mehreren Leiterplatten (428B) angeordnet ist und umgekehrt, wenn die erste der mehreren Leiterplatten über die zweite der mehreren Leiterplatten gefaltet ist, wodurch ein optimaler Volumenverbrauch in den mehreren Leiterplatten erreicht wird.

4. Verkapselungskonfiguration (520) für Elektronikkomponenten in einer flexiblen implantierbaren medizinischen Vorrichtung nach Anspruch 1, ferner umfassend:
eine flache Leiterplatte (532), die mehrere Elektronikkomponenten (534) umfasst,
wobei die flache Leiterplatte eine im Allgemeinen rechteckige Form aufweist;
wobei die mehreren Elektronikkomponenten auf beiden Seiten der flachen Leiterplatte positioniert sind;
wobei die größeren der mehreren Elektronikkomponenten näher an der Mitte der flachen Leiterplatte positioniert sind; und
wobei die kürzere der mehreren Elektronikkomponenten näher an den Kanten der flachen Leiterplatte positioniert ist, wodurch ein optimaler Volumenverbrauch in der flachen Leiterplatte erreicht wird,
wobei eine erste der mehreren Leiterplatten (524) mit der flachen Leiterplatte (532) mit einem Verbindungskabel (536) gekoppelt ist, und
wobei die Verkapselungskonfiguration eine zylindrische Form aufweist.

5. Verkapselungskonfiguration nach Anspruch 4, wobei die mehreren Leiterplatten und die flache Leiterplatte jeweils einen Durchmesser von gleich oder kleiner als 11 Millimeter aufweisen.

6. Verkapselungskonfiguration nach Anspruch 4, wobei die mehreren Leiterplatten und die flache Leiterplatte zusammen eine Gesamtlänge von gleich oder kleiner als 5 Zentimeter aufweisen.

## Revendications

1. Configuration d'encapsulation (420) pour composants électroniques dans un dispositif médical implantable flexible, comprenant :
une pluralité de circuits imprimés (422), chaque circuit imprimé de ladite pluralité de circuits imprimés comprenant au moins un composant électronique (426) ; et
une pluralité de câbles de connexion plats (424),
**caractérisée en ce que** :
chaque circuit imprimé de ladite pluralité de circuits imprimés présente une forme généralement circulaire ;
chaque câble de ladite pluralité de câbles de connexion plats couple électriquement des circuits imprimés adjacents de ladite pluralité de circuits imprimés alternativement à leurs extrémités opposées ; et
chaque circuit imprimé de ladite pluralité de circuits imprimés est replié sur un circuit imprimé adjacent de ladite pluralité de circuits imprimés de manière plissée, ce qui procure à ladite configuration d'encapsulation une forme en accordéon.

2. Configuration d'encapsulation selon la revendication 1, dans laquelle chacun desdits au moins un composants électroniques est positionné spécifiquement sur un circuit imprimé respectif de ladite pluralité de circuits imprimés en fonction de sa hauteur, ce qui permet d'obtenir une occupation de volume optimale dans ladite pluralité de circuits imprimés.

3. Configuration d'encapsulation selon la revendication 1, dans laquelle au moins un composant électronique (430₂) relativement haut d'un premier circuit imprimé de ladite pluralité de circuits imprimés (428A) est placé de façon complémentaire sur au moins un composant électronique (432₂) relativement bas d'un deuxième circuit imprimé de ladite pluralité de circuits imprimés (428B) et vice-versa quand ledit premier circuit imprimé de ladite pluralité de circuits imprimés est replié sur ledit deuxième circuit imprimé de ladite pluralité de circuits imprimés, ce qui permet d'obtenir une occupation de volume optimale dans ladite pluralité de circuits imprimés.

4. Configuration d'encapsulation (520) pour des composants électroniques dans un dispositif médical implantable flexible selon la revendication 1, comprenant en outre :
un circuit imprimé plat (532) comprenant une pluralité de composants électroniques (534),
dans laquelle ledit circuit imprimé plat présente une forme généralement rectangulaire ;
dans laquelle les composants de ladite pluralité de composants électroniques sont positionnés des deux côtés dudit circuit imprimé plat ;
dans laquelle les composants plus hauts de ladite pluralité de composants électroniques sont positionnés plus près du centre dudit circuit imprimé plat ; et
dans laquelle les composants plus bas de ladite pluralité de composants électroniques sont positionnés plus près des bords dudit circuit imprimé plat, ce qui permet d'obtenir une occupation de volume optimale dans ledit circuit imprimé plat,
dans laquelle un premier circuit imprimé de ladite pluralité de circuits imprimés (524) est couplé audit circuit imprimé plat (532) via un câble de connexion (536), et
dans laquelle ladite configuration d'encapsulation présente une forme cylindrique.

5. Configuration d'encapsulation selon la revendication 4, dans laquelle ladite pluralité de circuits imprimés et ledit circuit imprimé plat présentent chacun un diamètre inférieur ou égal à 11 millimètres.

6. Configuration d'encapsulation selon la revendication 4, dans laquelle ladite pluralité de circuits imprimés et ledit circuit imprimé plat présentent conjointement une longueur totale inférieure ou égale à 5 centimètres.
